# EUROPÄISCHE PATENTSCHRIFT

(11) **EP 2 063 922 B1**
(45) Veröffentlichungstag und Bekanntmachung des Hinweises auf die Patenterteilung: **11.07.2012**
(21) Anmeldenummer: 07820006.0
(22) Anmeldetag: 31.08.2007
(51) Int. Cl.: A61L 27/30, A61L 27/46, A61L 27/56, A61L 27/60, A61L 27/32, A61L 27/44

(54) **STRUKTURIERTE BESCHICHTUNGEN FÜR IMPLANTATE SOWIE VERFAHREN ZU DEREN HERSTELLUNG**
STRUCTURED COATINGS FOR IMPLANTS AND METHOD FOR PRODUCTION THEREOF
REVÊTEMENTS STRUCTURÉS POUR IMPLANTS ET LEUR PROCÉDÉ DE FABRICATION

(30) Priorität: 01.09.2006 DE 102006041023
(43) Veröffentlichungstag der Anmeldung: 03.06.2009
(73) Patentinhaber: Biocer-Entwicklungs-GmbH, 95447 Bayreuth (DE)
(72) Erfinder: HEIDENAU, Frank, 91257 Pegnitz (DE); ZIEGLER, Günter, 90541 Nürnberg (DE)
(74) Vertreter: Isarpatent
(86) Internationale Anmeldenummer: PCT/EP2007/059115
(87) Internationale Veröffentlichungsnummer: WO 2008/025840

(56) Entgegenhaltungen:
- EP-A- 1 882 722
- WO-A-2004/026346
- WO-A-2004/103421
- DE-A1-102004 012 411
- HOFFMANN B ET AL: "Corrosion behaviour, metal release and biocompatibility of implant materialscoated by TiO2-sol gel chemistry" BIOMEDIZINISCHE TECHNIK, FACHVERLAG SCHIELE UND SCHOEN GMBH. BERLIN, DE, Bd. 50, Nr. 10, 1. Januar 2005 (2005-01-01), Seiten 320-329, XP001538222 ISSN: 0013-5585
- HEIDENAU F ET AL: "A novel antibacterial titania coating: Metal ion toxicity and in vitro surface colonization" JOURNAL OF MATERIALS SCIENCE: MATERIALS IN MEDICINE, KLUWER ACADEMIC PUBLISHERS, BO, Bd. 16, Nr. 10, 1. Oktober 2005 (2005-10-01), Seiten 883-888, XP019212089 ISSN: 1573-4838

## Beschreibung

Die vorliegende Erfindung betrifft ein Verfahren zur Herstellung einer pulvergefüllten, keramischen Beschichtung auf einem Implantat sowie ein beschichtetes Implantat.

### Hintergrund der Erfindung und Stand der Technik

Die Bioakzeptanz und die Biokompatibilität eines Implantats hängen entscheidend von den Oberflächeneigenschaften des Implantats ab. Zum einen ist die Struktur der Oberfläche oder die Oberflächenmorphologie für das Anwachsen neuer Zellen an das Implantat entscheidend. Zum anderen kann das Zellwachstum gewünschter Zellen durch die Freisetzung von Wirkstoffen gefördert werden, oder das Wachstum unerwünschter Zellverbände, wie beispielsweise eingeschleppter Bakterien, die zu einer Infektion oder gar zu einem Abstoßen des Implantats führen können, gehemmt werden.

Daher wurden im Stand der Technik Beschichtungen auf Basis eines Sol-Gel-Verfahrens entwickelt, die Wirkstoffe freisetzen. Diese Wirkstofffreisetzung kann beispielsweise einen antibakteriellen Effekt besitzen, wie in der DE 10243132 beschrieben. Die Anwendung ist jedoch auf Metallionen beschränkt, deren organische Salze sich in einem Sol lösen lassen.

In einem Versuch, die Struktur eines Implantats an die natürlichen Gegebenheiten anzupassen, beschreibt die WO 2004/103421 A1 ein Knochenersatzmaterial in Form eines Granulats, das über ein Sol-Gel-Verfahren mit einem Calciumphosphatpulver hergestellt wird. Dieses Granulat kann auf ein Implantat beschichtet werden, muss jedoch über ein aufwendiges Verfahren, beispielsweise Plasmasprayverfahren, aufgebracht werden.

In der Anmeldung DE 10 2004 012411 A1 der Fa. DOT Coating wird ein ähnliches Material beschrieben. Eine Applikation als Schichtsystem mit einer definierten Oberflächenstrukturierung ist nicht beschrieben. Ebenso wenig ist eine gezielte Strukturierung der Oberfläche möglich. Zudem enthält das Material ein Kompositmaterial auf der Basis von Polykieselsäuren, das einen Anteil eines organischen Polymers aufweist. Dieses Material besitzt jedoch den Nachteil, dass es aufgrund der organischen Bestandteile eine verringerte Lagerstabilität aufweist und mit der in der Medizintechnik üblichen Methode der Sterilisation durch γ-Bestrahlung nur schwer sterilisiert werden kann. Eine Nachsterilisation in der Klinik ist ebenfalls erschwert, da ein Erhitzen des Materials auf 132 °C zu einer Degradation der organischen Bestandteile führt. Auch kann ein Wirkstoff, wie beispielsweise ein Antibiotikum, oder ein Polymer, die in dem Material vorliegen, durch Radikale, die durch γ-Bestrahlung entstehen, beschädigt oder immobilisiert werden.

Die DE 102 43 132 B4 offenbart eine antimikrobielle Titanoxidbeschichtung für Implantate, die mittels eines Sol-Gel-Verfahrens hergestellt wird. Bei dieser Anmeldung liegt das verwendete Metallsalz homogen und vollständig gelöst in der Sol-Gel-Matrix vor und nicht als Feststoffpartikel, wodurch eine Strukturierung der Oberfläche nicht möglich ist.

Die DE 600 22 197 T2 offenbart ein Material beispielsweise in Form von Partikeln, die über ein Sol-Gel-Verfahren hergestellt werden. Eine Zugabe von Partikeln während der Durchführung des Sol-Gel-Verfahrens ist nicht erwähnt. Zudem muss das Material zur Verfestigung auf Temperaturen von 800-1500°C erhitzt werden.

Eine einfache Sol-Gel-Beschichtung ohne Pulverfüllung ist aus der Publikation von M. Jokinen et al., J. Biomed. Mater. Res., 42 (1998) 295-302 bekannt.

Schließlich ist aus der Publikation von M. Teller et al., Key Engineering Materials, Vols. 284-286 (2005), Seiten 415-418, ein poröses Material bekannt, das mit Antibiotika beladen werden kann, um einen antimikrobiellen Körper herzustellen. Aufgrund der hohen Porosität des Materials ist es für eine Beschichtung eines Implantats nicht geeignet.

Alle oben genannten Verfahren besitzen den Nachteil, dass eine gezielte Strukturierung der Oberfläche und eine Anpassung der Wirkstoffabgabe nicht möglich ist.

Der vorliegenden Erfindung liegt daher die Aufgabe zugrunde, ein Verfahren zum Herstellen einer Beschichtung bereitzustellen, das ein gezieltes Einstellen der Oberflächenstruktur oder -morphologie einer Beschichtung auf einem Substrat ermöglicht, wobei gleichzeitig eine gezielte Einstellung einer Wirkstoffabgabe möglich ist.

### Zusammenfassung

Diese Aufgabe wird durch ein Verfahren zur Herstellung einer partikelgefüllten, metalloxidischen Beschichtung auf einem Implantat mit den Schritten:
a. Bereitstellen einer Mischung eines organischen Lösungsmittels und eines metallorganischen Precursors;
b. Versetzen der Mischung aus Schritt a. mit einem partikelförmigen Füllstoff, um eine Zubereitung herzustellen, wobei der partikelförmige Füllstoff eine Partikelgrösse im Bereich von 0,01 bis 10 µm besitzt
c. Aufbringen der unter b. hergestellten Zubereitung auf ein Implantat, um eine Beschichtung auf dem Implantat bereitzustellen; und
d. Trocknen der aufgebrachten Beschichtung,
wobei der partikelförmige Füllstoff beim Herstellen der Zubereitung in Schritt b. einen Anteil von 15 Gew.-%, bezogen auf die Summe der Masse an Precursormaterial und Lösungsmittel der Mischung aus Schritt a. nicht übersteigtgelöst.

### Ausführliche Beschreibung der Erfindung

Erfindungsgemäß wird ein Verfahren zur Herstellung einer gefüllten, biokompatiblen Metalloxid-Beschichtung auf einem Implantat bereitgestellt, mit dem ein Implantat herstellbar ist. Aus dieser Beschichtung können unter physiologischen Bedingungen aus der Beschichtung und/oder dem Füllstoff Wirkstoffe in die Umgebung abgegeben werden.

Gemäß einem ersten Aspekt stellt die vorliegende Erfindung ein Verfahren zur Herstellung einer partikelgefüllten, metalloxidischen Beschichtung auf einem Implantat mit den Schritten:
a. Bereitstellen einer Mischung eines organischen Lösungsmittels und eines metallorganischen Precursors;
b. Versetzen der Mischung aus Schritt a. mit einem partikelförmigen Füllstoff, um eine Zubereitung herzustellen, wobei der partikelförmige Füllstoff eine Partikelgrösse im Bereich von 0,01 bis 10 µm besitzt;
c. Aufbringen der unter b. hergestellten Zubereitung auf ein Implantat, um eine Beschichtung auf dem Implantat bereitzustellen; und
d. Trocknen der aufgebrachten Beschichtung,
wobei der partikelförmige Füllstoff beim Herstellen der Zubereitung in Schritt b. einen Anteil von 15 Gew.-%, bezogen auf die Summe der Masse an Precursormaterial und Lösungsmittel der Mischung aus Schritt a. nicht übersteigt bereit.

Das erfindungsgemäße Verfahren zur Beschichtung von Substraten, nämlich von Implantaten, weist die folgenden Schritte auf. Zunächst wird eine Mischung hergestellt, die als eine dünnflüssige Suspension, ein sogenanntes Sol, enthaltend ein organisches Lösungsmittel, eine metallorganische Verbindung sowie wahlweise Wasser und/oder eine Säure, vorliegt. Diese Mischung wird mit einem Füllmaterial versetzt.

Teilweise wird zur feindispersen Verteilung der Füllstoffe ein Dispergiermittel zugesetzt. Dieses Dispergiermittel kann beispielsweise ein Alkylolammoniumsalz niedermolekularer Polycarbonsäuren, ein Copolymer mit sauren Gruppen, eine Lösung von Copolymeren mit pigmentaffinen Gruppen, ein Acrylatcopolymer mit pigmentaffinen Gruppen, ein hochmolekulares BlockCopolymer mit pigmentaffinen Gruppen oder eine Lösungen von Acrylatcopolymeren mit basischen pigmentaffinen Gruppen sein.

Unter Sol ist erfindungsgemäß eine kolloidale Lösung zu verstehen, in der ein fester Stoff in feinster, d.h. im Wesentlichen in molekularer bzw. atomarer, Verteilung (Durchmesser der Solteilchen < 30 nm, bevorzugt < 10 nm) ohne Aggregatbildung in einem flüssigen Medium dispergiert ist. Erfindungsgemäß sind die metallorganischen Verbindungen in dem Sol bevorzugt vollständig gelöst. Das Sol kann auch als Nanosuspension bezeichnet werden, da die metallorganischen Verbindungen im Nanometerbereich verteilt vorliegen. Die aus dem Sol über einen Sol-Gel-Prozess entstehende oxidische Beschichtung wird auch als Matrix oder oxidische Matrix bezeichnet.

Unter Füllstoff ist dabei erfindungsgemäß eine Beimengung in partikulärer Form zu Verstehen, die entweder als strukturgebendes Element wirkt und/oder durch Abgabe von Wirkstoffen eine therapeutische Wirkung erzielt. Die Füllung kann unter physiologischen Bedingungen stabil oder abbaubar sein. Als Füllmaterialien kommen Metalle, Metalllegierungen, Gläser, Keramiken, anorganische Verbundwerkstoffe, anorganische Werkstoffverbunde, schwerlösliche Salze, insbesondere Kristalle schwerlöslicher Salze, oder Kombinationen aus diesen Materialien zum Einsatz.

Die partikelförmigen Füllstoffe können dicht, porös oder gradiert porös, d.h. mit einem Porositätsgradienten, sein. Es ist auch möglich, dass die Füllstoffe teilweise resorbierbar sind.

Beispiele für Metalle und Metalllegierungen, die erfindungsgemäß bevorzugt als Füllstoff eingesetzt werden können, sind elementare Metalle wie Silber, Kupfer, Calcium, Magnesium oder Zink, und Metalllegierungen, wie beispielsweise Messing.

Beispiele für keramische Materialien oder Keramiken, die erfindungsgemäß bevorzugt als Füllstoffe eingesetzt werden können, sind Oxide bzw. Nitride wie Aluminiumoxid, Zirkonoxid, Titanoxid, Siliziumoxid, Calciumphosphate, z.B. Hydroxylapatit, sowie Gläser und Glaskeramiken, bevorzugt Gläser oder Glaskeramiken, die sich unter physiologischen Bedingungen lösen oder auflösen.

Unter Wirkstoff sind sowohl Ionen der Elemente, aus denen sich der Füllstoff zusammensetzt, aber auch physikalisch oder chemisch an den Füllstoff gebundene oder in den Wirkstoff eingekapselte organische oder anorganische therapeutisch wirksame Mittel zu verstehen. Beispiele für Ionen sind Calcium-, Zink-, Silber-, Kupfer- oder Magnesium-Ionen. Daneben können Partikel eingesetzt werden, die diese Ionen enthalten oder freisetzen können.

Der Wirkstoff kann auch aus der oxidischen Matrix freigesetzt werden. Dazu kann beispielsweise ein Metallion in Form eines Metallsalzes zu dem Sol zugegeben werden, das homogen in der Lösung verteilt wird. Bei Aufbringen des Sols auf ein Substrat werden die Metallionen in die Matrix eingebaut und unter physiologischen Bedingungen wieder abgegeben. Derartige Metallsalze werden zusätzlich zu den Füllstoffen eingebracht.

Unter Implantat ist erfindungsgemäß ein Substrat zu verstehen, das dafür geeignet ist, in einen Patienten, d.h. im Bereich der Humanmedizin, oder einem Tier, d.h. im Bereich der Veterinärmedizin, implantiert zu werden. Beispiele für Implantate sind Katheter, Osteosynthesematerial, Endoprothesen, Fixateurs externes/internes, Nägel, Schrauben und/oder Drähte, Herzklappen, künstliche Blutgefäße und Shunts, gesichtschirurgische/plastische Implantate, Mittelohrimplantate, Dentalimplantate, etc.

Unter physiologischen und pathophysiologischen Bedingungen sind erfindungsgemäß Bedingungen zu verstehen, die in der Umgebung eines in einen Patienten implantierten Implantats vorherrschen können. Der Begriff umfasst erfindungsgemäß sämtliche Körperflüssigkeiten, die mit einem solchen implantierten Implantat in Kontakt kommen, aber auch sonstige Pufferlösungen, welche als Ersatz für Körperflüssigkeiten eingesetzt werden, wie z.B. eine physiologische Kochsalzlösung, Phosphatgepuffertes Kochsalz (PBS), Spüllösungen, Desinfektionslösungen und ähnliches.

Bevorzugt wird durch das Verfahren eine rein anorganische Beschichtung bereitgestellt. Diese anorganische Beschichtung ist frei von organischen Materialien, wie beispielsweise organischen Lösemitteln oder organischen Polymerverbindungen. Möglicherweise in den Beschichtungen enthaltene organische Materialien können über thermisches Erhitzen, beispielsweise während eines Brennvorgangs, entfernt werden.

Die Dicke der erfindungsgemäßen Beschichtung liegt im Bereich von einigen Hundert Nanometern, bevorzugt ca. 50 bis 10000 nm, bevorzugter 100 - 5000 nm, noch bevorzugter 150 - 1500 nm. Durch die besondere Dünne der Schicht und der geringen Beladung der Schicht mit Füllmaterial wird die Anhaftung der Schicht an dem Substrat bedeutend verbessert.

Die Größe der Füllstoffpartikel soll einen Maximalwert nicht überschreiten. Die Matrix, die aus dem metallorganischen Precursor bzw. Vorläufer entsteht, wirkt wie ein Klebstoff, der das Substrat bedeckt und die Füllstoffpartikel umhüllt. Die Größe der Füllstoffe soll den fünffachen Wert, bevorzugt den dreifachen Wert, der Dicke der Matrix nicht überschreiten. Wird die Größe der Partikel größer als der fünffache Wert der Dicke der Matrix, dann wird die resultierende Beschichtung nicht mehr ausreichend auf dem Substrat fixiert und kann sich unter Belastung ablösen.

Durch das Verhältnis von Partikelgröße und Schichtdicke der Matrix kann die Oberflächenrauheit gezielt eingestellt werden. Die Füllstoffpartikel ragen bei einem derartigen Verhältnis von Füllstoffpartikel zu Matrixdicke aus der Beschichtung heraus und bilden eine unregelmäßige, im Mikro-Bereich raue Oberfläche. Bevorzugt liegen die Rₐ-Werte für die Oberflächen im Bereich von 0,01 bis 10 µm, bevorzugt 0,1 bis 5 µm, weiter bevorzugt 0,2 bis 1,5 µm. Diese raue Oberfläche kann von Zellen besser besiedelt werden als die üblicherweise glatten oder kugelgestrahlten Oberfläche von Implantaten.

Durch das Einbringen von partikelförmigen Füllstoffen kann die Oberfläche strukturiert werden. Diese Strukturierung kann in Form einer Mikrorauheit, beispielsweise mit den oben genannten Oberflächenrauheiten, durch die Partikelgröße, die Partikelgrößenverteilung und die Partikelmenge gezielt eingestellt werden.

Die Füllstoffpartikel besitzen eine Größe im Bereich von 0,01 bis 10 µm, bevorzugter im Bereich von 0,1 bis 5 µm, und am bevorzugtesten im Bereich von 0,2 bis 1,5 µm. Die Größe der Füllstoffpartikel ist in einem engen Größenbereich verteilt. Dabei ist der d₅₀-Wert der Partikel bevorzugt kleiner 3,5 µm, bevorzugt kleiner oder gleich 3 µm, weiter bevorzugt kleiner oder gleich 2 µm. Der d₅₀-Wert gibt die Partikelgröße an, unter dessen Wert 50% aller Partikel liegen. Dadurch kann eine homogene Oberfläche mit einer gleichmäßigen Oberflächenrauheit erreicht werden.

Der Gehalt an Füllstoff liegt im Bereich von bis zu 15 Gew-%, bevorzugt im Bereich von 0,005-10 Gew.-%, und bevorzugter im Bereich von 0,01-8 Gew.-%, weiter bevorzugt im Bereich von 0,1-1 Gew.-%. Die Mengenangabe ist dabei auf die Summe der Masse an Precursormaterial und Lösungsmittel bezogen, die zusammen 100% entspricht.

Unter der Dicke der Matrix soll lediglich die Dicke der aus dem Sol-Gel-Verfahren resultierenden oxidischen Matrix verstanden werden. Die Dicke der gesamten Beschichtung umfasst dagegen auch die eingebrachten Partikel. Die Partikel werden in die Matrix eingebettet, die bevorzugt eine Dicke im Bereich von 20 bis 500 nm, bevorzugter im Bereich von 50 bis 300 nm, weiter bevorzugt im Bereich von 80 bis 200 nm besitzt. Dabei ist jedoch auch das Verhältnis der Größe der Partikel zur Dicke der Matrix, wie oben erwähnt, zu berücksichtigen.

Gemäß einer bevorzugten Ausgestaltung der Erfindung kann das Verfahren zur Beschichtung mehrfach durchgeführt werden. Dabei wird jeweils eine Schicht mit einer Dicke im oben genannten Bereich aufgetragen. Die Gesamtdicke der mehreren Schichten beträgt dann ein Vielfaches der Dicke der Einzelschichten, d.h., der Bereich der Dicke der Schichten beträgt dann ein Vielfaches der oben genannten Schichtdicke für eine einzelne Schicht. Die Füllstoffe können in den unterschiedlichen Schichten unterschiedlich oder gleich sein. Auch kann die Konzentration von Schicht zu Schicht variieren, um so einen Gradienten bei der Abgabe von Wirkstoffen zu erreichen. Es ist auch möglich, die unterschiedlichen Schichten so mit unterschiedlichen Wirkstoffen zu beladen, dass beispielsweise zu Beginn ein antimikrobiell wirkender Wirkstoff abgegeben wird, und nach einer vorher definierten Zeitspanne ein knochenwachstumsfördernder Wirkstoff freigesetzt wird. So kann die Wirkstoffabgabe zu unterschiedlichen Zeitpunkten auf die unterschiedlichen Bedürfnisse individuell angepasst werden.

Als Implantate können erfindungsgemäß metallische Implantate, Implantate aus Metalllegierungen, Kunststoffe, Gläser, keramische Implantate, Verbundwerkstoffe, Knochenersatzmaterialien oder Kombinationen aus diesen verwendet werden. Beispiele für bevorzugte Implantate sind Katheter, Osteosyntheseplatten, Endoprothesen, Fixateurs externes/internes, Nägel, Schrauben und/oder Drähte, Herzklappen, künstliche Blutgefäße und Shunts, gesichtschirurgische/plastische Implantate, Mittelohrimplantate und Dentalimplantate.

Beispiele für Metalle und Metalllegierungen, die erfindungsgemäß bevorzugt als Implantate eingesetzt werden können, sind Titan, Stahl, Eisen und/oder Stahl-, Eisen-, Titanlegierungen, bevorzugt TiAl6V4 oder TiAl6Nb7, Cobalt-Chrom-Basislegierungen und/oder Osteosynthesestahl, bevorzugt AISI316L, sowie elementare Metalle wie Silber, Kupfer, Calcium, Magnesium oder zink.

Beispiele für Kunstoffe, die erfindungsgemäß bevorzugt als Implantate eingesetzt werden können, sind Polymere wie Polyethylen, Polypropylen, Polytetrafluorethylen, Polyethylenterephthalat, Polyamide, Polyurethane, Polysiloxane, Polysiloxan-Elastomere, Polyetheretherketon und Polysulfon sowie Polysaccharide und Polylactide.

Beispiele für keramische Materialien, die erfindungsgemäß bevorzugt als Implantate eingesetzt werden können, sind Oxide bzw. Nitride wie Aluminiumoxid, Zirkonoxid, Titanoxid, Siliziumoxid, Calciumphosphate, z.B. Hydroxylapatit, sowie Gläser und Glaskeramiken, bevorzugt Gläser oder Glaskeramiken, die sich unter physiologischen Bedingungen lösen oder auflösen.

Als Knochenersatzmaterialien können bevorzugt Autografts, Allografts, Xenografts oder synthesische Knochenersatzmaterialien eingesetzt werden.

Das nahezu wasserflüssige Gemisch, das den Füllstoff enthält, wird auf das Substrat aufgebracht. Dies kann durch Tauchbeschichtung, Spin-Coaten, Rakeln, Drucken oder Aufsprühen oder andere Verfahren gemäß dem Stand der Technik geschehen.

Die in dem Gemisch enthaltenen Precursoren sind bevorzugt mehrfach koordinierte, metallorganische Verbindungen mit Liganden -OR, die über das Sauerstoffatom an das Metall koordiniert sind, wobei R ein linearer oder verzweigter Alkylrest mit einer bevorzugten Kettenlänge von C2 bis C8 ist. An Stelle oder zusätzlich können ungesättigte Alkylreste (Alkenylreste) und/oder Sauerstoff- und/oder Stickstoff-haltige Alkylreste bzw. Alkenylreste erfindungsgemäß für spezielle Anwendungen, wie beispielsweise UV-Härtbarkeit, ebenfalls verwendet werden, die ebenfalls bevorzugt 2 bis 8 C-Atome, aber auch längere Alkylketten bis C-12, aufweisen. Beispiele für geeignete sauerstoffkoordinierte Alkylreste sind insbesondere Ethyl-, n-Propyl-, Isopropyl-, n-Butyl-, Isobutyl-, n-Pentyl- und/oder Isopentylreste.

Beispiele für geeignete Alkenylreste sind Acrylate, Methacrylate oder längerkettige Alkylketten oder verzweigte Alkylketten, die Doppelbindungen tragen. Die bevorzugte Kettenlänge der Hauptkette beträgt C2 bis C12, die der Seitenketten C2 bis C6.

Beispiele für geeignete O-substituierte und N-substituierte Alkyl- und/oder Alkenylreste sind kohlenstoffkettenbasierte Reste, die den bereits beschriebenen Anforderungen genügen, zusätzlich jedoch Ether-, Keto- oder Aminogruppen enthalten.

Beispiele für erfindungsgemäß verwendbare Precursoren sind Tetrabutoxytitanat, Titanisobutoxytitanat, Titantetrapropylat, Titantetraisopropylat, Titantetraacetylacetonat, Titantetraethoxytitanat oder die Analoga des Siliciums, Zirkons oder Aluminiums, soweit sie chemisch stabil sind. Unter Metalloxiden werden daher insbesondere die Oxide der oben genannten Metalle Titan, Silicium, Zirkon und Aluminium verstanden.

Als organisches Lösungsmittel werden bevorzugt lineare oder verzweigte Alkohole mit Kettenlängen von 2 bis 8 Kohlenstoffatomen verwendet, z.B. Ethanol, Propanol, Isopropylalkohol, n-Butanol, sec-Butanol oder Kombinationen aus den genannten Alkoholen, wobei Ethanol und n-Butanol besonders bevorzugt sind. Weitere organische Lösungsmittel, die erfindungsgemäß einsetzbar sind, sind cyclische, aromatische und/oder heteroaromatische Kohlenwasserstoffe oder deren Derivate, bspw. Cyclopentan, Cyclohexan, Benzol, Toluol, Tetrahydrofuran oder Dioxan, wobei Benzol, Toluol und/oder Tetrahydrofuran besonders bevorzugt sind. Das organische Lösungsmittel kann vom Fachmann entsprechend dem verwendeten Metallsalz oder der metallorganischen Verbindung gewählt werden. Erfindungsgemäß können auch beliebige Mischungen der oben genannten Lösungsmittel verwendet werden.

Wahlweise kann in der Zubereitung Wasser und/oder eine Säure, vorzugsweise eine mineralische Säure, enthalten sein.

Als mineralische Säure wird bevorzugt Salpetersäure verwendet. Es können jedoch zusätzlich oder stattdessen andere Säuren, wie Salzsäure, Schwefelsäure, Phosphorsäure, oder organische Säuren wie Zitronensäure, Trifluoressigsäure oder Essigsäure eingesetzt werden.

Bei Verwendung einer Säure beträgt die Konzentration der Säure bevorzugt 1 bis 50 Mol-% des eingesetzten metallorganischen Precursors, bevorzugter 2 bis 20 Mol-%, noch bevorzugter 8 bis 10 Mol-%.

Die Konzentration des Lösungsmittels beträgt bevorzugt das 5 bis 50-fache der Molmenge des metallorganischen Precursors, bevorzugter das 15 bis 40-fache, noch bevorzugter das 20 bis 35-fache.

Bei oder nach dem Aufbringen der oben beschriebenen Zubereitung, vorzugsweise in Form eines Sols, auf das Substrat kann erfindungsgemäß erreicht werden, dass dieses durch Abdampfen des Lösungsmittels und/oder durch Einstellung von stöchiometrischen Eduktverhältnissen in ein verfestigtes formbeständiges aber ein leicht deformierbares System bzw. ein Gel übergeht, wobei die Füllstoffe homogen dispergiert in dem verfestigten System oder Gel vorliegen.

Anschließend erfolgt eine Trocknung, wonach die beschichteten Implantate direkt verwendet werden können. Die Trocknung kann unter Sauerstoff-, Stickstoff-, Argon- oder Luftatmosphäre erfolgen. Bevorzugt wird die Trocknung im Bereich von Raumtemperatur (25°C) bis 100°C, weiter bevorzugt von Raumtemperatur bis 50°C durchgeführt. Das Trocknen kann bei Normaldruck oder vermindertem Druck (Unterdruck) erfolgen. Es ist auch möglich, das Trocknen durch ein Gefriertrocknungsverfahren oder unter überkritischen Trocknungsbedingungen zu bewirken. Die nachfolgende Wärmebehandlung erfolgt zur mechanischen Stabilisierung bzw. zur Verdichtung der Beschichtungen. Z.B. kann durch das Erhitzen bei etwa 500°C bevorzugt eine Keramisierung der Beschichtung erfolgen. Bei Kunststoffimplantaten erfolgt bevorzugt ein weniger starkes Erhitzen.

Es kann sich eine Erwärmung der Beschichtung auf 100 - 1000°C, bevorzugt bei 200 bis 800°C, noch bevorzugter bei 400 bis 600°C anschließen. Diese Erwärmung kann separat zum Trocknen oder zusammen mit dem Trocknen erfolgen. Die verweildauer des Substrats mit der aufgebrachten Beschichtung ist dabei nicht so entscheidend wie die Temperatur selbst. Die Verweildauer kann beispielsweise von der Art des Substrats und dem Verhalten des Substrats bei Erhitzen abhängen. Robustere Substrate, wie beispielsweise Keramiken, können schneller erhitzt und wieder abgekühlt werden, als beispielsweise Metalle, die sich beim Erhitzen stärker ausdehnen. Entscheidend ist jedoch die erreichte Temperatur, da bei Überschreiten von Grenztemperaturen die Matrix eine Umwandlung erfährt.

Wahlweise erfolgt der Trocknungsschritt unter überkritischen Bedingungen, bevorzugt in einem Autoklaven. Unter "überkritischen Bedingungen", wie sie hierin genannt sind, ist bei festgelegtem Autoklavenvolumen ein Druck-Temperatur-Zeit Profil zu verstehen, bei welchem das verwendete Lösungsmittel ohne Ausbildung einer Phasengrenze durch Dichteerniedrigung vom flüssigen in den gasförmigen Zustand jenseits des physikalisch definierten kritischen Punktes gebracht wird und so aus der Schicht entfernt wird.

Die besonderen Vorteile dieses Verfahrens liegen in der Beibehaltung der geltypischen, nanometerskaligen Porenstruktur und damit der Schaffung einer sehr hohen spezifischen Oberfläche der Beschichtung. Dadurch ist es einerseits möglich, die Ionenfreisetzungskinetik aus den Füllstoffen zusätzlich zu beeinflussen und andererseits durch die Schaffung einer strukturierten, porösen Oberfläche das Wachstum von Körperzellen wie z.B. Osteoblasten oder Fibroblasten, positiv zu beeinflussen.

Durch das erfindungsgemäße Verfahren ist es möglich, durch Verdünnung bzw. Mehrfachbeschichtung, die Konzentration an e-luierten Wirkstoffen aus der Beschichtung genau einzustellen. Durch Mehrfachbeschichtung kann die Wirkung der Beschichtung verstärkt werden, da dann eine größere Menge Wirkstoffe bereitgestellt werden kann. Bevorzugt ist eine Ein- und/oder eine Zweifachbeschichtung.

Eine Mehrfachbeschichtung wird erfindungsgemäß hergestellt, indem die oben genannten Schritte a bis d, d.h. Bereitstellen der Mischung, Herstellen der Zubereitung, Aufbringen der hergestellten Zubereitung auf ein Implantat und Trocknen der aufgebrachten Beschichtung,
ein oder mehrmals wiederholt werden, so dass eine oder mehrere zusätzliche Beschichtungen auf dem Implantat erzeugt werden. Wahlweise kann jeweils nach der Durchführung der vorgenannten Verfahrensschritte oder einmalig am Ende ein Erhitzen auf 100 bis 1000°C durchgeführt werden.

Auch bei einer Mehrfachbeschichtung sind die Füllstoffe bzw. Wirkstoffe in den einzelnen Beschichtungen jeweils homogen verteilt.

Im Folgenden werden einige Beispiele beschrieben, die jedoch den Umfang der Erfindung nicht einschränken sollen In den Beispielen wird auf folgende Figuren Bezug genommen:

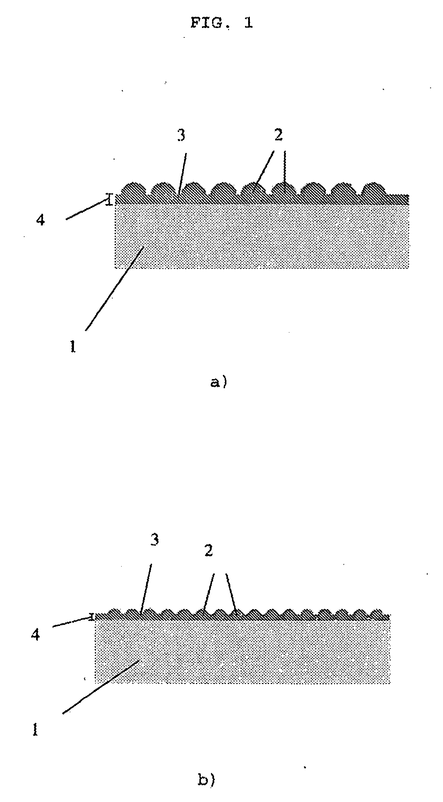

Fig. 1 zeigt die schematische Darstellung des Prinzips der Beschichtung. Durch Einsatz von Füllstoffen unterschiedlicher Partikelgröße bzw. in unterschiedlicher Menge kann eine gezielte Einstellung der Oberflächenmorphologie bzw. eine gezielte Wirkstofffreisetzung erreicht werden. Fig. 1 zeigt ein Substrat 1 mit einer darauf aufgebrachten oxidischen Matrix 3, in der Füllstoffpartikel 2 eingelagert sind. Der Durchmesser der Füllstoffpartikel überschreitet dabei nicht das Fünffache der Dicke 4 der oxidischen Matrix. Durch die unterschiedliche Größe der Füllstoffpartikel kann eine unterschiedliche Oberflächenrauheit eingestellt werden, wie im vergleich zwischen den Figuren 1a) und 1b) ersichtlich ist.

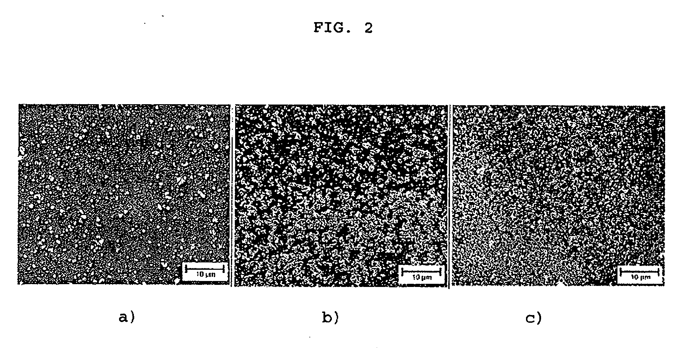

Fig. 2 zeigt elektronenmikroskopische Aufnahmen von beschichteten Oberflächen, die unterschiedliche Mengen an Calciumphosphat-Pulver (Hydroxylapatit) enthalten. Die Bilder zeigen Füllstoffgehalte von 1 (Fig. 1a), 3 (Fig. 1b) und 8 Gew% (Fig. 1c) bezogen auf die Gesamtmasse an vorgelegtem Sol, die gemäß Beispiel 1 hergestellt wurden.

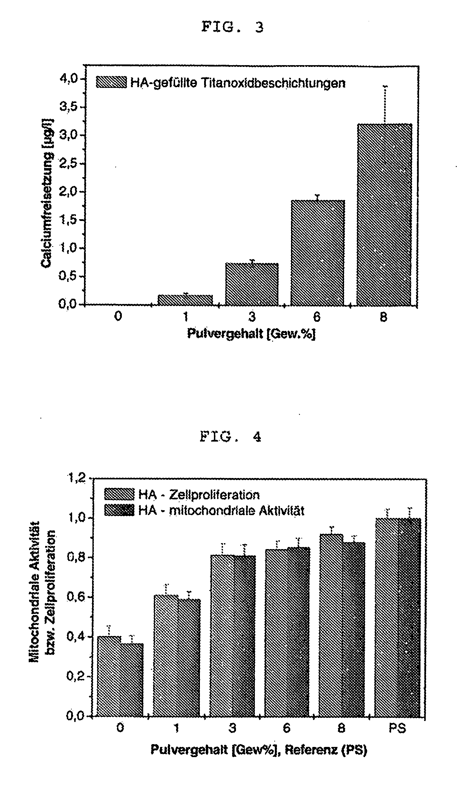

Fig. 3 zeigt die Freisetzung an Calciumionen unter physiologischen Bedingungen aus Beschichtungen, die mit unterschiedlichen Mengen an Calciumphosphat-Pulver (Hydroxylapatit) gefüllt wurden (vgl. Fig. 2). Als Referenzmaterial (auf 100% gesetzt) wurde das Polystyrol (PS) der Zellkulturschalen verwendet.
Fig 4. zeigt die Zellzahlentwicklung von fetalen Mausosteoblasten (MC3T3-E1) nach 48 h Kultur auf Beschichtungen, die mit unterschiedlichen Mengen an Calciumphosphat-Pulver (Hydroxylapatit) gefüllt wurden (vgl. Fig. 2).

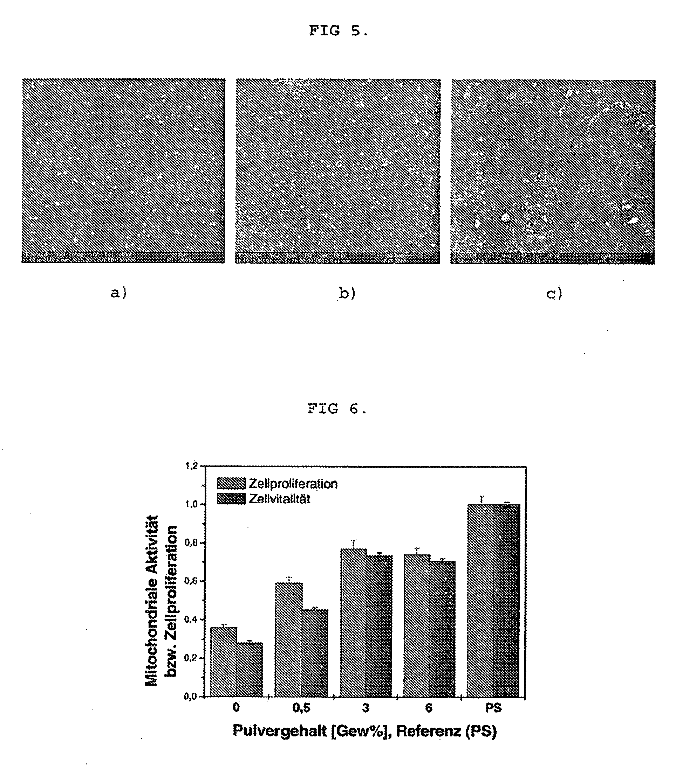

Fig. 5 zeigt in den Figuren 5a) bis 5c) elektronenmikroskopische Aufnahmen von beschichteten Oberflächen, die unterschiedliche Mengen an Titanoxid-Pulver enthalten. Die Bilder zeigen Füllstoffgehalte von 0,5 (Fig. 5a), 3 (Fig. 5b) und 6 Gew% (Fig. 5c) bezogen auf die Gesamtmasse an vorgelegtem Sol.
Fig 6. zeigt die Zellzahlentwicklung und die mitochondriale Aktivität von fetalen Mausosteoblasten (MC3T3-E1) nach 48h Kultur auf Beschichtungen, die mit unterschiedlichen Mengen an Titanoxid-Pulver gefüllt wurden (vgl. Fig. 5).

### Beispiele

### Beispiel 1: Beschichtung mit Füllstoff Hydroxylapatit

69,5 g Tetrabutoxytitanat wurden in 500 g n-Butanol bei RT gelöst und 2 h gerührt. Dann wurde, bezogen auf die Gesamtmasse des vorgelegten Sols (569,5 g), 17,1 g (entsprechend 3 Gel.-%) kommerzielles Calciumphosphatpulver (Fa. Merck, D₅₀ < 2µm) portionsweise zugegeben. Zur Dispergierung wurde 1 Gew.-% eines Dispergiermittels (DB194, wasserfrei; Fa. Byk Chemie, Wesel) zugegeben und weitere 2h gerührt.

Die anschließende Beschichtung erfolgte durch Tauchen des Probekörpers aus Glas mit einer Abzugsgeschwindigkeit von 1,5 mm/s. Anschließend wurde bei Raumtemperatur 1h getrocknet und die Beschichtung bei 500°C über 10 min keramisiert (calciniert). Eine elektronenmikroskopische Aufnahme der Beschichtung ist in Fig. 2b) gezeigt.

Entsprechend der obigen Beschreibung wurden zubereitungen mit 1 Gew.-% und 8 Gew.-% Calciumphosphatpulver anstelle von 3 Gew.-% Calciumphosphatpulver hergestellt und auf Glas aufgebracht. Die Figuren 2a) und 2c) zeigen elektronenmikroskopische Aufnahmen der Beschichtungen mit 1 Gew.-% bzw. 8 Gew.-% Calciumphosphatpulver.

Gemäß oben angegebenem Beispiel 1 wurden Beschichtungen auf TiAl6V4, Titan, einem Edelstahl und dem Kunststoff Polymethylmethacrylat (PMMA) aufgebracht. Bei der Beschichtung des PMMA entfiel der Keramisierungsschritt. Anstelle dessen wurde nach dem Trocknen 1 h bei 120 °C getempert.

### Beispiel 2: Beschichtung mit Füllstoff Titanoxid

41,7 g Tetrabutoxytitanat wurden in 300 g n-Butanol bei RT gelöst und 2 h gerührt. Dann wurde, bezogen auf die Gesamtmasse des vorgelegten Sols (341,7 g), 1,71g (entsprechend 0,5 Gew%), 10,26 g (entsprechend 3 Gew.-%), 20,52 g (entsprechend 6 Gew.-%) kommerzielles Titanoxidpulver (Fa. Alfa Aesar, Karlsruhe; D₅₀: 0,3 µm) portionsweise zugegeben. Zur Dispergierung wurde 1 Gew.-% eines Dispergiermittels (DB194, wasserfrei; Fa. Byk Chemie, Wesel) zugegeben und weitere 2h gerührt.

Die anschließende Beschichtung erfolgte durch Tauchen des Probekörpers aus Glas mit einer Abzugsgeschwindigkeit von 1,5 mm/s. Anschließend wurde bei Raumtemperatur 1h getrocknet und die Beschichtung bei 500°C über 10 min keramisiert.

Gemäß oben angegebenem Beispiel 2 wurden Beschichtungen auf TiAl6V4, Titan, einem Edelstahl, Zirkonoxid und dem Kunststoff Polymethylmethacrylat (PMMA) aufgebracht. Bei der Beschichtung des PMMA entfiel der Keramisierungsschritt. Anstelle dessen wurde nach dem Trocknen 1 h bei 120 °C getempert.

### Beispiel 3: Darstellung der Wirkungsweise

Um die Wirkungsweise der pulvergefüllten Beschichtung zu demonstrieren wurden Glassubstrate (Scheiben, Durchmesser 14,5 mm) mit Beschichtungen gemäß Beispiel 1 versehen, die unterschiedliche Mengen an kommerziellem Calciumphosphatpulver (Fa. Merck, D₅₀ < 2µm) enthalten. Es wurde einerseits die Calciumfreisetzung unter physiologischen Bedingungen bestimmt und andererseits das Zellwachstum auf den erhaltenen Oberflächen in Abhängigkeit von der Pulverfüllung.

### Bestimmung der Calciumkonzentration:

Die Bestimmung der Calciumkonzentration erfolgte mittels eines photometrischen Tests. Dieser beruht auf der Komplexbildung von o-Cresolphtalein Complexon (CPC, Fa. Fluka) mit Calcium im alkalischen pH-Bereich. Die violette Färbung des sich bildenden Farbkomplexes wird bei 575 nm absorptiv quantifiziert. Das CPC Reagent setzt sich wie folgt zusammen. 625 mg 8-Hydroxyquinolin (Fa. Fluka) wurden in 25 ml Dimethylsulfoxid (Fa. Serva) gelöst_{;} anschließend 10 mg o-CPC und 250 µl 36 % HCl (Fa. Fluka) zugegeben und mit 250 ml Aqua dest. aufgefüllt. Für den Diethylaminpuffer wurden in 40 ml Aqua dest. 2 ml Diethylamin und 25 mg KCl gelöst und auf 50 ml mit Aqua dest aufgefüllt. Für den Test wurden 50 µl Probe, Standard sowie Blindwert (reiner Lysepuffer) in 2,5 ml PS Mikroküvetten (Fa. Brand) vorgelegt und 960 µl Aqua dest. zugegeben. Im Anschluss wurden je 1 ml CPC-Reagent und 1 ml Diethylaminpuffer zupipettiert, gemischt und nach 5 min Inkubation im Spektrophotometer (Fa. Beckmann Coulter) gemessen. Zur Ermittlung der Konzentration diente eine Calciumstandardreihe.

### Versuchsbedingungen des Zelltests:

*Kulturmedium:* 90% RPMI 1640 (= 2,05mM glutaminhaltiges Serum), 10% FKS (fötales Kälberserum); Inkubation: 48h, 37°C, 5% CO2, statische Kultur, Dunkelheit).
*Zell-Linien: MC3T3-E1 (fetale Mausosteoblasten)*
Kultur: 24-Well Kulturschalen, Polystyrol
*Inokulum:* 120.000 Zellen/ml und Well (Vertiefung), lg-Phase

*Zellproliferation:* Trypsinierung (300 µl Trypsin-EDTA) für die Dauer von 8 Minuten im Schüttelinkubator bei 37°C, Abstoppen der Enzymreaktion mit 700 µl Kulturmedium. Bestimmung der Zellzahl im Coulter-Counter.
*Mitochondriale* Aktivität: WST-Test (Fa. Roche, Mannheim)

Es ist aus Figur 3 ersichtlich, dass proportional zur Pulverfüllung auch die Menge an freigesetztem Calcium steigt. Hierdurch kann auch gezeigt werden, dass trotz der mechanisch festen Einbindung der Füllstoffe in die Matrix ein Austausch mit dem umgebenden Medium statt findet und Wirkstoffe durch die Sol-Gel-Matrix hindurch wirksam werden.

Die Gegenüberstellung der Calciumionenfreisetzung (Fig. 3) und der Zelluntersuchungen (Fig. 4) zeigen deutlich eine proportionale Abhängigkeit des Zellwachstums zur Calciumfreisetzung aus der Beschichtung. Im Bereich ab 6 Gew% Pulvergehalt ist eine Sättigung erreicht und das Zellwachstum wird nicht weiter angeregt. Der Übergang in ein Plateau demonstriert aber, dass der Effekt rein auf den freigesetzten Calciumionen und nicht auf der Morphologieänderung durch die unterschiedlichen Pulvermengen beruht, da sonst ein signifikanter Anstieg der Zellzahlen zwischen 6 und 8 Gew% Füllstoff beobachtet werden müsste.

Fig. 6 zeigt die Entwicklung der Zellzahl und der mitochondrialen Aktivität in Abhängigkeit von der eingesetzten Pulvermenge. Da bei dem Füllstoff Titanoxid keine Wirkstoffreisetzung erfolgt wird das unterschiedliche Zellwachstum erfindungsgemäß durch die Änderung der Oberflächenmorphologie bewirkt. Die unterschiedliche Oberflächenbeschaffenheit wird in FIG. 5 dargestellt. Da bei dem Füllstoff Titanoxid bei 6 Gew% Füllgrad keine homogene Schichtbildung mehr möglich ist, verschlechtern sich entsprechend die Zellzahl und die mitochondriale Aktivität gegenüber den Proben, die nur mit 3 Ges%, Pulver gefüllt wurden.

## Patentansprüche

1. Verfahren zur Herstellung einer partikelgefüllten, metalloxidischen Beschichtung auf einem Implantat mit den Schritten:
a. Bereitstellen einer Mischung eines organischen Lösungsmittels und eines metallorganischen Precursors;
b. Versetzen der Mischung aus Schritt a. mit einem partikelförmigen Füllstoff, um eine Zubereitung herzustellen, wobei der partikelförmige Füllstoff eine Partikelgrösse im Bereich von 0,01 bis 10 µm besitzt;
c. Aufbringen der unter b. hergestellten Zubereitung auf ein Implantat, um eine Beschichtung auf dem Implantat bereitzustellen; und
d. Trocknen der aufgebrachten Beschichtung,
wobei der partikelförmige Füllstoff beim Herstellen der Zubereitung in Schritt b. einen Anteil von 15 Gew.-%, bezogen auf die Summe der Masse an Precursormaterial und Lösungsmittel der Mischung aus Schritt a. nicht übersteigt.

2. Verfahren nach Anspruch 1,
**dadurch gekennzeichnet,**
**dass** die Mischung zusätzlich Wasser und/oder eine mineralische Säure, bevorzugt Salpetersäure, Salzsäure, Schwefelsäure, Phosphorsäure, eine organische Säure, bevorzugt Essigsäure, Trifluoressigsäure oder Zitronensäure, oder deren Gemische, enthält, und/oder
**dass** die Mischung zusätzlich ein Dispergiermittel enthält.

3. Verfahren nach einem oder mehreren der vorhergehenden Ansprüche,
**dadurch gekennzeichnet,**
**dass** das Lösungsmittel ein linearer oder verzweigter C₂-C₈ Alkohol, bevorzugt Methanol, Ethanol, n-Propanol, isoPropanol, n-Butanol, iso-Butanol und tert.-Butanol, oder ein cyclischer, aromatischer oder heteroaromatischer Kohlenwasserstoff, bevorzugt Benzol oder Toluol, oder deren Mischungen, ist.

4. Verfahren nach einem oder mehreren der vorhergehenden Ansprüche,
**dadurch gekennzeichnet,**
**dass** der metallorganische Precursor eine metallorganische Verbindung mit einem mehrfach koordinierten Metallatom ist, wobei Liganden -OR über das Sauerstoffatom an das Metallatom koordiniert sind und R ein C₂-C₈ Alkyl oder Alkylenrest ist, der wahlweise mit einem oder mehreren Sauerstoff- und/oder Stickstoffatomen unterbrochen und/oder substituiert sein kann, wobei
der metallorganische Precursor insbesondere ausgewählt ist aus der Gruppe, bestehend aus Tetraethoxytitanat, Tetra(n-propoxy)titanat, Tetra(iso-propoxy)titanat, Tetra(n-butoxy)titanat, Tetra(iso-butoxy)titanat, Tetra(tert.-butoxy)titanat, Tetraethoxyorthosilikat, Tetra(n-butoxy)orthosilikat, Tetra(iso-butoxy)orthosilikat und Tetra(tert.-butoxy)orthosilikat,
wobei das Metallatom des metallorganischen Precursors insbesondere ausgewählt ist aus den Elementen Titan, Silizium, Aluminium und Zirkonium.

5. Verfahren nach einem oder mehreren der vorhergehenden Ansprüche,
**dadurch gekennzeichnet,**
**dass** der Füllstoff aus Metallen, bevorzugt Kupfer oder Silber, Metalllegierungen, bevorzugt Messing, Kunststoffen, Gläsern und Glaskeramiken, bevorzugt unter physiologischen Bedingungen lösliche Gläser und Glaskeramiken, Keramiken, bevorzugt Titanoxid oder Calciumphosphat, insbesondere bevorzugt Hydroxylapatit, Verbundwerkstoffen, schwerlöslichen Salzen und/oder Kombinationen aus diesen Materialien besteht.

6. Verfahren nach einem oder mehreren der vorhergehenden Ansprüche,
**dadurch gekennzeichnet,**
**dass** der Füllstoff unter physiologischen Bedingungen einen Wirkstoff abgeben kann, wobei
der Wirkstoff insbesondere ein Ion ist, bevorzugt ein Calcium-, Zink-, Silber-, Kupfer- oder Magnesium-Ion, oder ein therapeutisch wirksames Mittel, bevorzugt ein Antibiotikum ist.

7. Verfahren nach einem oder mehreren der vorhergehenden Ansprüche,
**dadurch gekennzeichnet,**
**dass** das Implantat ein Katheter, eine Osteosyntheseplatte, eine Endoprothese, ein Fixateur externe, ein Fixateur interne, ein Nagel, eine Schraube oder ein Draht, eine Herzklappe, ein künstliches Blutgefäß oder ein Shunt, ein gesichtschirurgisches/plastisches Implantat, ein Mittelohrimplantat oder ein Dentalimplantat ist, und/oder dass das Implantat aus einem Metall, einer Metalllegierung, einem Glas, einer Keramik, einem Kunststoff, einem Verbundwerkstoff oder einem Knochenersatzmaterial besteht, wobei
das Metall oder die Metalllegierung insbesondere Titan, Stahl, Eisen, eine Stahl-, Eisen-, Titan- und/oder CoCr-Legierung ist, bevorzugt TiAl6V4 oder TiAl6Nb7, ein Osteosynthesestahl, bevorzugt AISI316L, ist, oder der Kunststoff Polyethylen, Polypropylen, Polytetrafluorethylen, Polyethylenterephthalat, Polyamide, Polyurethane, Polysiloxane, Polysiloxan-Elastomere, Polyetheretherketon, Polysulfon, Polysaccharid und/oder Polylactid ist.

8. Verfahren nach einem oder mehreren der vorhergehenden Ansprüche,
**dadurch gekennzeichnet,**
**dass** das Aufbringen durch Tauchbeschichtung, Spin-Coaten, Rakeln, Drucken oder Aufsprühen erfolgt, und/oder das Trocken an Luft, in einer Inertgasatmosphäre, bevorzugt einer Stickstoff- oder Edelgasatmosphäre, oder unter überkritischen Trocknungsbedingungen erfolgt.

9. Verfahren nach einem oder mehreren der vorhergehenden Ansprüche,
**dadurch gekennzeichnet,**
**dass** nach Schritt d. in einem weiteren Schritt e. die aufgebrachte Beschichtung auf 100 bis 1000°C, bevorzugt auf 200 bis 800°C, und am bevorzugtesten auf 400 bis 600°C erhitzt wird.

10. Verfahren nach einem oder mehreren der vorhergehenden Ansprüche,
**dadurch gekennzeichnet,**
**dass** die Zubereitung aus Schritt b. in Form eines Sols aufgebracht wird, wobei das Sol, in welchem der Füllstoff homogen verteilt ist, bei oder nach dem Auftragen in Schritt c. in ein Gel übergeht, in welchem der Füllstoff homogen verteilt vorliegt, und/oder
**dass** die Zubereitung aus Schritt b. mit einer solchen Schichtdicke aufgebracht wird, dass die Schichtdicke nach dem Trocknen in Schritt d. und wahlweise dem Erhitzen in Schritt e. 50-1000 nm, bevorzugt 50-500 nm, noch bevorzugter 150-300 nm beträgt.

11. Verfahren nach einem oder mehreren der vorhergehenden Ansprüche,
**dadurch gekennzeichnet,**
**dass** die Schritte b. bis d. und wahlweise Schritt e. mehrfach durchgeführt werden,
wobei gegebenenfalls der Füllstoffgehalt der Zubereitung nach Schritt b. jeweils so variiert wird, dass die unterschiedlichen Beschichtungen unterschiedliche Füllstoffkonzentrationen aufweisen, und
wobei gegebenenfalls die unterschiedlichen aufgebrachten Beschichtungen unterschiedliche Füllstoffe aufweisen.

12. Implantat mit einer Beschichtung, herstellbar nach dem Verfahren gemäß einem oder mehreren der vorhergehenden Ansprüche, wobei
gegebenenfalls aus den in der Beschichtung enthaltenen Füllstoffen unter physiologischen Bedingungen Wirkstoffe in das umgebende Medium herauslösbar sind.

13. Implantat nach Anspruch 12,
**dadurch gekennzeichnet,**
**dass** die Schichtdicke 50-1000 nm, bevorzugt 50-500 nm, noch bevorzugter 150-300 nm beträgt.

14. Implantat nach einem oder mehreren der Ansprüche 12 - 13,
**dadurch gekennzeichnet,**
**dass** die Füllstoffe in einer Beschichtung jeweils homogen verteilt sind, wobei
die in der Beschichtung enthaltenen Füllstoffe insbesondere Calcium-, Zink-, Silber-, Kupfer-, Magnesium-Ionen enthalten.

15. Implantat nach einem oder mehreren der Ansprüche 12 - 14, **dadurch gekennzeichnet, dass** das Implantat ein Katheter, eine Osteosyntheseplatte, eine Endoprothese, ein Fixateur externe, ein Fixateur interne, ein Nagel, eine Schraube oder ein Draht, eine Herzklappe, ein künstliches Blutgefäß oder ein Shunt, ein gesichtschirurgisches/plastisches Implantat, ein Mittelohrimplantat oder ein Dentalimplantat ist.

## Claims

1. Process for preparing a particle-filled, metal oxide coating on an implant, comprising the following steps:
a. providing a mixture of an organic solvent and an organometallic precursor;
b. adding a particle-shaped filler to the mixture from step a. in order to prepare a preparation, the particle-shaped filler having a particle size between 0.01 and 10 µm;
c. applying the preparation prepared in step b. to an implant to provide a coating on the implant; and
d. drying the coating applied,
wherein the particle-shaped filler does not exceed a proportion of 15 % by weight, based on the total weight of precursor material and solvent of the mixture from step a., when preparing the preparation in step b.

2. Process according to claim 1, **characterised in that** the mixture additionally contains water and/or a mineral acid, preferably nitric acid, hydrochloric acid, sulphuric acid, phosphoric acid, an organic acid, preferably citric acid, trifluoroacetic acid or acetic acid, or mixtures thereof, and/or that the mixture further contains a dispersant.

3. Process according to one or more of the preceding claims, **characterised in that** the solvent is a linear or branched C₂-C₈ alcohol, preferably methanol, ethanol, n-propanol, isopropanol, n-butanol, isobutanol and tert-butanol, or a cyclic, aromatic or heteroaromatic carbohydrate, preferably benzene or toluene, or mixtures thereof.

4. Process according to one or more of the preceding claims, **characterised in that** the organometallic precursor is an organometallic compound with a multicoordinated metal atom, -OR ligands being coordinated to the metal atom by the oxygen atom and R being a C₂-C₈ alkyl or alkylene residue optionally interrupted and/or substituted by one or more oxygen and/or nitrogen atoms, whereat the organometallic precursor in particular is selected from the group consisting of tetraethoxy titanate, tetra(n-propoxy) titanate, tetra(isopropoxy) titanate, tetra(n-butoxy) titanate, tetra(isobutoxy) titanate, tetra(tert-butoxy) titanate, tetraethoxy orthosilicate, tetra(n-butoxy) orthosilicate, tetra(isobutoxy) orthosilicate and tetra(tert-butoxy) orthosilicate, whereat the metal atom of the organometallic precursor in particular is selected from the elements titanium, silicon, aluminium and zirconium.

5. Process according to one or more of the preceding claims, **characterised in that** the filler is formed from metals, preferably copper or silver, metal alloys, preferably brass, plastics materials, glass and glass ceramics, preferably glass and glass ceramics which dissolve under physiological conditions, ceramics, preferably titanium oxide or calcium phosphate, in particular preferably hydroxyl apatite, composite materials, sparingly soluble salts and/or combinations of these materials.

6. Process according to one or more of the preceding claims, **characterised in that** the filler may release an active substance under physiological conditions, whereat the active ingredient is an ion, preferably a calcium, zinc, silver, copper or magnesium ion, or a therapeutically active substance, preferably an antibiotic.

7. Process according to one or more of the preceding claims, **characterised in that** the implant is a catheter, an osteosynthesis plate, an endoprosthesis, an external fixator, an internal fixator, a nail, a screw or a wire, a heart valve, a synthetic blood vessel or a shunt, a facial surgery/plastic implant, a middle ear implant or a dental implant, and/or the implant is formed from a metal, a metal alloy, a glass, a ceramic, a plastics material, a composite material or a bone substitute material, whereat the metal or metal alloy in particular is titanium, steel, iron, a steel, iron, titanium and/or CoCr alloy, preferably TiAl6VA or TiAl6Nb7, an osteosynthesis steel, preferably AISI316L, or the plastics material is polyethylene, polypropylene, polytetrafluoroethylene, polyethylene teraphthlate, polyamides, polyurethanes, polysiloxanes, polysiloxane elastomers, polyetheretherketone, polysulphone, polysaccharide and/or polylactides.

8. Process according to one or more of the preceding claims, **characterised in that** application is carried out by dip coating, spin coating, application by doctor blade, printing or spraying, and/or the drying process is carried out in air, in an inert gas atmosphere, preferably a nitrogen or noble gas atmosphere, or under supercritical drying conditions.

9. Process according to one or more of the preceding claims, **characterised in that**, after step d., the coating applied is heated to between 100 and 1000°C, preferably between 200 and 800°C, and most preferably between 400 and 600°C, in a further step e.

10. Process according to one or more of the preceding claims, **characterised in that** the preparation from step b) is applied in the form of a sol, the sol, in which the filler is dispersed homogeneously, changing into a gel, in which the filler is homogeneously dispersed, during or after the application process in step c), and/or the preparation from step b. is applied so as to have a layer thickness such that the layer thickness after the drying procedure in step d. and the optional heating procedure in step e. is between 50 and 1000 nm, preferably between 50 and 500 nm, more preferably between 150 and 300 nm.

11. Process according to one or more of the preceding claims, **characterised in that** the steps b. to d. and optionally step e. are carried out a plurality of times, and whereat, if applicable, the filler content of the preparation according to step b. is in each case varied in such a way that the different coatings contain different concentrations of fillers, and whereat if applicable the different coatings applied contain different fillers.

12. Implant with a coating which can be produced according to the process according to one or more of the preceding claims, whereat if applicable, active substances contained in the coating can be eluted into the surrounding medium under physiological conditions.

13. Implant according to claim 12, **characterised in that** the layer thickness is between 50 and 1000 nm, preferably between 50 and 500 nm, and more preferably between 150 and 300 nm.

14. Implant according to one or more of claims 12 and 13, **characterised in that** the fillers in each coating are homogeneously dispersed, whereat the fillers contained in the coating in particular contain calcium, zinc, silver, copper, magnesium ions.

15. Implant according to one or more of claims 12-14, **characterised in that** the implant is a catheter, an osteosynthesis plate, an endoprosthesis, an external fixator, an internal fixator, a nail, a screw or a wire, a heart valve, a synthetic blood vessel or a shunt, a facial surgery/plastic implant, a middle ear implant or a dental implant

## Revendications

1. Procédé pour produire sur un implant un revêtement en oxyde métallique chargé de particules, comportant les étapes suivantes :
a. préparer un mélange d'un solvant organique et d'un précurseur organométallique,
b. ajouter au mélange résultant de l'étape a. une charge sous forme de particules pour obtenir une préparation, la taille des particules de la charge étant comprise entre 0,01 et 10 µm,
c. appliquer sur un implant la préparation produite en b. pour obtenir un revêtement sur l'implant et
d. sécher la préparation appliquée,
lorsqu'on produit la préparation à l'étape b., la charge sous forme de particules ayant un pourcentage pondéral qui ne dépasse pas 15 % si on se réfère à la somme obtenue en ajoutant la masse de matériau précurseur et le solvant du mélange qui résulte de l'étape a.

2. Procédé selon la revendication 1, **caractérisé en ce que** le mélange contient en outre de l'eau et/ou un acide minéral, de préférence l'acide nitrique, l'acide chlorhydrique, l'acide sulfurique, l'acide phosphorique, un acide organique de préférence l'acide acétique, l'acide trifluoracétique ou l'acide citrique, ou leur mélange, et/ou
que le mélange contient en outre un agent dispersant.

3. Procédé selon une ou plusieurs des revendications précédentes, **caractérisé en ce que** le solvant est un alcool C₂-C₈ linéaire ou ramifié, de préférence le méthanol, l'éthanol, le n-propanol, l'iso-propanol, le n-butanol, l'iso-butanol et le tert.-butanol ou un hydrocarbure cyclique, aromatique ou hétéro-aromatique, de préférence le benzol ou le toluol ou leur mélange.

4. Procédé selon une ou plusieurs des revendications précédentes, **caractérisé en ce**
**que** le précurseur organométallique est un composé organométallique comportant un atome métallique à plusieurs coordinations, les ligands -OR étant coordonnés à l'atome métallique par l'atome d'oxygène et R est un alkyle C₂-C₈ ou un radical alkyle qui peut être interrompu et/ou substitué à volonté par un ou plusieurs atomes d'oxygène et/ou d'azote,
le précurseur organométallique étant en particulier choisi parmi le groupe constitué par le tétraéthoxytitanate, le tétra(n-propoxy)titanate, le tétra(isopropoxy)titanate, le tétra(n-butoxy)titanate, le tétra(iso-butoxy)titanate, le tétra(tert.-butoxy)titanate, le tétraéthoxyorthosilicate, le tétra(n-butoxy)orthosilicate, le tétra(iso-butoxy)orthosilicate et le tétra(tert.-butoxy)orthosilicate,
l'atome métallique du précurseur organométallique étant en particulier choisi parmi le groupe constitué par les éléments suivants : titane, silicium, aluminium et zirconium.

5. Procédé selon une ou plusieurs des revendications précédentes, **caractérisé en ce que**
la charge est de préférence un métal, de préférence le cuivre ou l'argent, des alliages métalliques, de préférence le laiton, les matières synthétiques, le verre et les vitrocéramiques, de préférence les verres et les vitrocéramiques solubles dans des conditions physiologiques, les céramiques, de préférence l'oxyde de titane ou le phosphate de calcium, en particulier de préférence l'hydroxylapatite, les matériaux composites, les sels peu solubles et/ou des combinaisons de ces matériaux.

6. Procédé selon une ou plusieurs des revendications précédentes, **caractérisé en ce que**,
dans des conditions physiologiques, la charge peut libérer un principe actif,
le principe actif étant en particulier un ion, de préférence un ion calcium, zinc, argent, cuivre ou magnésium ou un agent à effet thérapeutique, de préférence un antibiotique.

7. Procédé selon une ou plusieurs des revendications précédentes, **caractérisé en ce**
**que** l'implant est un cathéter, une plaque d'ostéosynthèse, une endoprothèse, un fixateur externe, un fixateur interne, un clou, une vis ou un fil métallique, une valvule de coeur, un vaisseau sanguin artificiel ou une dérivation, un implant plastique ou de chirurgie faciale, un implant de l'oreille moyenne ou un implant dentaire et/ou
**que** l'implant est en métal, en alliage métallique, en verre, en céramique, en matériau synthétique, en matériau composite ou en matériau de substitution osseuse,
le métal ou l'alliage métallique étant de préférence le titane, l'acier, le fer, un alliage d'acier, de fer, de titane et/ou de CoCr, de préférence TiAl6V4 ou TiAl6Nb7, un acier d'ostéosynthèse, de préférence AISI316L, ou le matériau synthétique étant choisi parmi les matériaux suivants : polyéthylène, polypropylène, polytétrafluoréthylène, polyéthylènetéréphthalate, polyamide, polyuréthane, polysiloxane, élastomères polysiloxane, polyétheréthercétone, polysulfone, polysaccharide et/ou polylactide

8. Procédé selon une ou plusieurs des revendications précédentes, **caractérisé en ce**
**que** l'application a lieu par revêtement par immersion, revêtement par centrifugation, raclage, pression ou pulvérisation et/ou le séchage à l'air, dans une atmosphère de gaz inerte, de préférence une atmosphère d'azote ou de gaz rare ou dans des conditions surcritiques de séchage.

9. Procédé selon une ou plusieurs des revendications précédentes, **caractérisé en ce**
**que**, suite à l'étape d., le revêtement appliqué est chauffé au cours d'une autre étape e. jusqu'à une température comprise entre 100 et 1 000 °C, de préférence entre 200 et 800 °C, tout particulièrement entre 400 et 600 °C.

10. Procédé selon une ou plusieurs des revendications précédentes, **caractérisé en ce**
**que** la préparation résultant de l'étape b. est appliquée sous forme d'un sol, le sol dans lequel la charge est réparti de manière homogène se transformant pendant ou après l'application à l'étape c. en gel dans lequel la charge est répartie de manière homogène et/ou que la préparation résultant de l'étape b. est appliquée en une couche d'une épaisseur telle que, après le séchage à l'étape d. et éventuellement le chauffage à l'étape e., elle est comprise entre 50 et 1 000 nm, de préférence entre 50 et 500 nm, tout particulièrement entre 150 et 300 nm.

11. Procédé selon une ou plusieurs des revendications précédentes, **caractérisé en ce**
**que** les étapes b. à d. et éventuellement l'étape e sont réalisées plusieurs fois,
où le cas échéant la teneur en charge de la préparation après l'étape b. est à chaque fois modifiée de telle manière que les différents revêtements présentent différentes concentrations en charge
et les différents revêtements appliqués ayant le cas échéant différentes charges.

12. Implant comportant un revêtement, qui peut être produit par le procédé selon une ou plusieurs des revendications précédentes,
dans lequel des principes actifs peuvent le cas échéant, dans des conditions physiologiques, diffuser dans le milieu environnant à partir des charges que contient le revêtement.

13. Implant selon la revendication 12, **caractérisé en ce que** la couche a une épaisseur de 50 à 1 000 nm, de préférence de 50 à 500 nm, encore mieux de 150 à 300 nm.

14. Implant selon une ou plusieurs des revendications 12 et 13, caractérisé en ce ue les charges sont toujours réparties de façon homogène dans un revêtement,
les charges que contient le revêtement contenant en particulier des ions calcium, zinc, argent, cuivre, magnésium.

15. Implant selon une ou plusieurs des revendications 12 à 14, **caractérisé en ce**
**que** l'implant est un cathéter, une plaque d'ostéosynthèse, une endoprothèse, un fixateur externe, un fixateur interne, un clou, une vis ou un fil métallique, une valvule de coeur, un vaisseau sanguin artificiel ou une dérivation, un implant plastique ou de chirurgie faciale, un implant de l'oreille moyenne ou un implant dentaire.
